Europäisches Patentamt

European Patent Office

Office européen des brevets

(19)

(11) Publication number: 0 314 163
A2

(12) **EUROPEAN PATENT APPLICATION**

(21) Application number: 88118000.4

(22) Date of filing: 28.10.88

(51) Int. Cl.⁴: **C07D 471/04** , //(C07D471/04, 243:00,221:00)

(30) Priority: 30.10.87 IT 4855787

(43) Date of publication of application:
03.05.89 Bulletin 89/18

(84) Designated Contracting States:
AT BE CH DE ES FR GB GR IT LI LU NL SE

(71) Applicant: DOMPE' FARMACEUTICI S.p.A.
Via S. Martino, 12-12/A
I-20122 Milano(IT)

(72) Inventor: Giani, Roberto
via P.Nenni, 58
Locate Triulzi(IT)
Inventor: Parini, Ettore
via Roma, 153
Cologno Monzese Milano(IT)
Inventor: Tonon, Giancarlo
via Beatrice d'Este, 7
Milano(IT)
Inventor: Borsa, Massimiliano
via Carlo Porta, 2
Vimodrone Milano(IT)

(74) Representative: Beneduce, Gianna
Via Poggibonsi, 14
I-20146 Milano(IT)

(54) Process for the preparation of 6,11-dihydro-5H-pyrido(2,3-b)(1,5)benzodiazepin-5-one.

(57) The present invention is concerned with a process for the preparation of 6,11-dihydro-5H-pyrido-[2,3-b][1,5]benzodiazepin-5-one by the condensation of 2-chloronicotinic acid and o-phenylenediamine in a cyclic organic solvent at an elevated temperature, in which the reaction of condensation is carried out at a temperature of 100 to 190°C in a cycloalkanol or a mixture of cycloalkanols as [a] cyclic organic solvent(s). The end product is in pure state and it may be utilized as such.

EP 0 314 163 A2

## Process for the preparation of 6,11-dihydro-5H-pyrido[2,3-b][1,5]benzodiazepin-5-one

The present invention is concerned with an improved method for the preparation of 6,11-dihydro-5H-pyrido[2,3-b][1,5]benzodiazepin-5-one.

6,11-Dihydro-5H-pyrido[2,3-b][1,5]-bezodiazepin-5-one is encompassed in a class of heterocyclic compounds, many of which possessing pharmacological activity, described in Bull. Soc. Chem. France, 1966, N° 7, page 2 316 and it is prepared by condensing under reflux in chlorobenzene, 2-chloronicotinic acid and 0-phenylenediamine.

From the reaction mixture, adding ethanol as a precipitation aid, 6,11-dihydro-5H-pyrido[2,3-b]-[1,5]benzodiazepin-5-one was separated in a very raw state in the form of a product of greenish yellow color, melting at 280° C, and therefore for obtaining a product of sufficient purity it was necessary to carry out several re-crystallizations from suitable solvents. Examples of such solvents are acetic acid, pyridine and dioxane. Using as solvents tetrahydronaphthalene, dichloro- or trichlorobenzene or glycol as described in DE-OS 24 24 811 this did not result in improving the quality of the end product, because even making use of these solvents, the so obtained 6,11-dihydro-5H-pyrido[2,3-b][1,5]benzodiazepin-5-one still was very impure.

6,11-Dihydro-5H-pyrido[2,3-b][1,5]-benzodiazepin-5-one is the starting material in the preparation of 11-(1-methylpiperidin-4-carbonyl)-6,11-dihydro-5H-pyrido[2,3-b][1,5]benzodiazepin-5-one, a very interesting anti-ulcer compound, which has been described in USP 4,556,653; the carrying out of a process which allows to obtain the starting material 6,11-dihydro-5H-pyrido[2,3][1,5]-benzodiazepin-5-one in a purer state and in higher yield, represents therefore an aim of great interest.

Hence the problem underlying to the invention is to create a process for the preparation fo 6,11-dihydro-5H-pyrido[2,3-b][1,5]benzodiazepin-5-one by the condensation of 2-chloronicotinic acid and o-phenylenediamine by which this is obtained in a purer state and in a higher yield without the necessity to carry out re-crystallizations.

Surprisingly this has been attained by the invention.

Surprisingly now it has been found that in the preparation of 5,11-dihydro-5H-pyrido[2,3][1,5]-benzodiazepin-5-one, when the condensation between 2-chloronicotinic acid and o-phenylenediamine takes place in a cycloalkanol(s), the final product is obtained in a high degree of purity (yellow color, melting point 295 to 296° C) and no further re-crystallizations of the same are necesary.

Hence the subject-matter of the invention is a process for the preparation of 6,11-dihydro-5H-pyrido[2,3-b][1,5]benzodiazepin-5-one by the condensation of 2-chloronicotinic acid and o-phenylenediamine in a cyclic organic solvent at an elevated temperature, which is characterized in that the reaction of condensation is carried out at a temperature of 100 to 190° C in a cycloalkanol or mixture of cycloalkanols as [a] cyclic organic solvent(s).

The duration of the reaction of condensation may vary upon the cycloalkanol used, but in any case it is comprised in the range of 3 to 14 hours.

Suitably as [a] cycloalkanol(s) such having from 3 to 8, preferably 4 to 6, more preferably 5 or 6, carbon atoms in the cycloalkane ring is/are used. The most preferred cycloalkanols are cyclopentanol and cyclohexanol, particularly cyclohexanol, and mixtures of such. Also cycloalkanols substituted by 1 or more alkyl group(s) having from 1 to 4, preferably 1 or 2 carbon atom(s) and/or further hydroxy group(s) in the cycloalkane ring are advantageous.

Preferably a temperature of 135 to 165° C is used. Particularly preferably the process according to the invention is carried out at the reflux temperature.

The product 6,11-dihydro-5H-pyrido[2,3-b][1,5]-benzodiazepin-5-one can be isolated by usual methods, such as precipitation, for example, the alkanols, such as ethanol, and filtration.

The use of the [a] cycloalkanol(s) as [a] solvent(s) in the above mentioned reaction makes it possible to result in an improved process by which 6,11-dihydro-5H-pyrido[2,3-b][1,5]benzodiazepin-5-one is obtained at a very high degree of purity so that it may be used as such without previous purification.

Furthermore by the process of the present invention a considerable improvement has been obtained concerning the total yield of the end product, this yield being at least 80%, whereas according to the process described in Bull. Soc. Chem. France, 1966, N° 7, page 2 316, the partial yield of the raw product, evaluated before the re-crystallization, was only 70%.

A further advantage of the process of the present invention is represented by the fact that in accordance with it, the use of re-crystallization solvents is no longer required, and therefore, apart from realizing noticeable savings, it is no longer necessary to have at disposal storage facilities suitable for those solvents, neither is it necessary to follow precise rules for their use, nor to have suitable plants for their depuration and re-use.

The example which follows is given for the purpose of illustrating the invention.

## Example

6,11-Dihydro-5H-pyrido[2,3-b][1,5]benzodiazepin-5-one

A 100 ml flask was charged with 15.7 g of 2-chloronicotinic acid, 10.8 g of o-phenylenediamine and 60 ml of cyclohexanol. The reaction mixture was refluxed for 13 hours and the water which formed, removed by azeotropic distillation. The reaction mixture was then cooled to 60°C and diluted with 40 ml of ethanol, stirring for 30 minutes at room temperature. The precipitate which formed was seperated by filtration and washed several times with ethanol. 16.9 g of a crystalline yellow product, consisting of 6,11-dihydro-5H-pyrido[2,3-b][1,5]benzodiazepin-5-one, melting at 295 to 296°C, were obtained.

## Claims

1.) A process for the preparation of 6,11-dihydro-5H-pyrido[2,3-b][1,5]benzodiazepin-5-one by the condensation of 2-chloronicotinic acid and o-phenylenediamine in a cyclic organic solvent at an elevated temperature, characterized in that the reaction of condensation is carried out at a temperature of 100 to 190°C in a cycloalkanol or a mixture of cycloalkanols as [a] cyclic organic solvent(s).

2.) A process according to claim 1, characterized in that cyclopentanol or cyclohexanol or a mixture of such is used as [a] cycloalkanol(s).

3.) A process according to claim 1 or 2, characterized in that a temperature of 135 to 165°C is used.

4.) A process according to claims 1 to 3, characterized in that it is carried out at the reflux temperature.